**Europäisches Patentamt**

(19) **European Patent Office**

**Office européen des brevets**

(11) Veröffentlichungsnummer: **0 084 094
B1**

(12) # EUROPÄISCHE PATENTSCHRIFT

(45) Veröffentlichungstag der Patentschrift:
**17.04.85**

(51) Int. Cl.⁴: **A 61 F 2/32**

(21) Anmeldenummer: **82110496.5**

(22) Anmeldetag: **13.11.82**

(54) Gelenkkopf für eine Kugelgelenkprothese.

(30) Priorität: **14.01.82 CH 197/82**

(43) Veröffentlichungstag der Anmeldung:
**27.07.83 Patentblatt 83/30**

(45) Bekanntmachung des Hinweises auf die Patenterteilung:
**17.04.85 Patentblatt 85/16**

(84) Benannte Vertragsstaaten:
**AT BE DE FR GB IT NL**

(56) Entgegenhaltungen:
**DE - A - 2 524 923
DE - A - 2 742 464
DE - A - 3 023 354
GB - A - 1 371 335**

(73) Patentinhaber: **GEBRÜDER SULZER
AKTIENGESELLSCHAFT, Zürcherstrasse 9,
CH-8401 Winterthur (CH)**
Patentinhaber: **Protek AG, Stadtbachstrasse 64,
CH-3001 Bern (CH)**

(72) Erfinder: **Müller, Maurice E., Prof. Dr.-med., Inselspital,
CH-3000 Bern (CH)**
Erfinder: **Niederer, Peter Gino, Dipl.-Ing.,
Reichenbachstrasse 6, CH-3052 Zollikofen (CH)**
Erfinder: **Frey, Otto, Walrütistrasse 56,
CH-8400 Winterthur (CH)**

(74) Vertreter: **Dipl.-Ing. H. Marsch Dipl.-Ing. K. Sparing
Dipl.-Phys.Dr. W.H. Röhl Patentanwälte,
Rethelstrasse 123, D-4000 Düsseldorf (DE)**

**Beschreibung**

Die Erfindung betrifft einen Gelenkkopf für eine Kugelgelenkprothese, der als Kugelschale ausgebildet ist und schirmartig auf einem Stamm sitzt, wobei zwischen der Innenwand der Kugelschale und dem Stamm ein freier Ringraum vorhanden ist.

Ein Gelenkkopf der vorstehend genannten Art ist beispielsweise aus der GB-A-1 371 335 bekannt; wird diese bekannte Gelenkkopfprothese mit einer künstlichen Gelenkpfanne zu einer Totalprothese vervollständigt, so haben sich infolge einer ungenügenden Schmierung der relativ zueinander bewegten Gleitflächen von Gelenkkopf und Gelenkpfanne unter Umständen Schwierigkeiten ergeben, die zu einem erhöhten Verschleiß und Abrieb an den genannten Gelenkflächen geführt haben.

Weiterhin ist es bekannt (DE-B-2 524 923 und DE-A-2 742 464) eine zentrale mit der Prothesenhalsachse fluchtende Bohrung durch den Gelenkkopf für den Transport von Körperflüssigkeit zur Schmierung der Gleitflächen von Gelenkkopf und Pfannenschale vorzusehen. Die Praxis hat gezeigt, daß die Schmierwirkung bei den bekannten Konstruktionen häufig ungenügend ist, insbesondere wenn der Transport der Körperflüssigkeit nicht — wie bei der erwähnten DE-B-2 524 923 vorgesehen — durch eine Pumpwirkung des axial verschiebbaren Zapfens unterstützt wird.

Aufgabe der Erfindung ist es, die Verteilung der als Schmiermittel wirkenden Körperflüssigkeit zwischen den Gleitflächen bei Konstruktionen der eingangs genannten Art mit schirmartig ausgebildeten Gelenkköpfen zu verbessern. Diese Aufgabe wird gemäß der Erfindung dadurch gelöst, daß im den Ringraum begrenzenden Bereich der Kugelschale über den Umfang verteilt Durchtrittsöffnungen vorgesehen sind, die an die Außenseite der Kugelschale führen.

Mit Hilfe von beispielsweise sechs über den Umfang verteilten Durchtrittsöffnungen kann die im Ringraum zwischen dem Stamm des Gelenkkopfes und seiner Kugelschale vorhandene Flüssigkeit besser und in größeren Mengen als bisher zwischen die Gleitflächen eindringen; sie wird durch diese Öffnungen darüber hinaus nahe den bei Belastungen besonders belasteten Flächenbereichen im zweiten Quadranten eines im Mittelpunkt der Kugelschale angenommenen Koordinatensystems zugeführt.

Die Durchtrittsöffnungen können bezüglich der Symmetrieachse der Kugelschale oder bezüglich des Kugelschalenmittelpunktes radial angeordnet sein; insbesondere ist es aber auch möglich, daß sie parallel zu der genannten Symmetrieachse verlaufen. Weiterhin hat es sich als zweckmäßig erwiesen, wenn die Kugelschale zusätzlich eine an sich bekannte zentrale Bohrung aufweist, die eine offene Verbindung zu einem Hohlraum im Innern der Konusbüchse herstellt; der Hohlraum der Büchse kann auf diese Weise als zusätzliches Schmiermittelreservoir und/ oder der Ablagerung für eventuell Abrieb dienen.

Im folgenden wird die Erfindung anhand von Ausführungsbeispielen im Zusammenhang mit der Zeichnung näher erläutert.

Fig. 1 zeigt schematisch einen Schnitt durch den Prothesenhals, die Kugelschale und die Gelenkpfanne einer in ein Becken implantierten Hüftprothese;

Fig. 2 ist eine Variante des in Fig. 1 gezeigten Gelenkkopfes.

Ein nur andeutungsweise skizzierter Verankerungsschaft 1 einer Hüftgelenkprothese, der zum Einsetzen in den Femur bestimmt ist, trägt an seinem oberen Ende einen konisch zulaufenden Zapfen 2. Auf diesen ist mit einem entsprechenden Mutterkonus 3 ein Gelenkkopf 4 aufgesetzt.

Der Gelenkkopf 4 besteht aus einer relativ dünnen, schirmartigen Kugelschale 5, an die als »Stiel« eine Konusbüchse 6 angesetzt ist. Die Konusbüchse 6 nimmt den Mutterkonus 3 auf, der bei einzelnen Gelenkköpfen 4 unterschiedlich tief in der Konusbüchse 6 sitzt, um — wie erwähnt — den Abstand, den die Kugelschale 5 in Richtung der Achse des Zapfens 2 von einer willkürlich gewählten Bezugslinie 7 hat, variieren zu können. Es ist jedoch auch ohne weiteres möglich, die Konusbüchse 6 durch einen massiven Stamm oder Stiel zu ersetzen, was nicht ausdrücklich gezeigt ist.

Der schirmartige Aufbau des Gelenkkopfes 4 ergibt im Innern der Kugelschale 5 einen freien Ringraum 8. Dieser steht in offener Verbindung mit einem Raum 10, der durch eine Gelenkkapsel 11 von der Umgebung mindestens weitgehend abgeschlossen und mit Körperflüssigkeit gefüllt ist; diese Gelenkkapsel 11, die bei der Operation geöffnet wird und sich im Laufe der Zeit wieder schließt, besteht aus einer narbengewebeartigen Haut, die mit dem nicht dargestellten Femurknochen einerseits und dem Beckenknochen 12 andererseits verwachsen ist.

In den Beckenknochen 12 seinerseits ist eine künstliche Gelenkpfanne 13 eingesetzt, die beispielsweise aus Polyäthylen besteht. Ihre Pfannenschale dient als Lagerfläche für den Gelenkkopf 4. Während der Bewegungen des Hüftgelenkes bewegt sich der Gelenkkopf 4 in der Pfannenschale, wobei die in der Gelenkkapsel eingeschlossene Flüssigkeit als Schmiermittel dient.

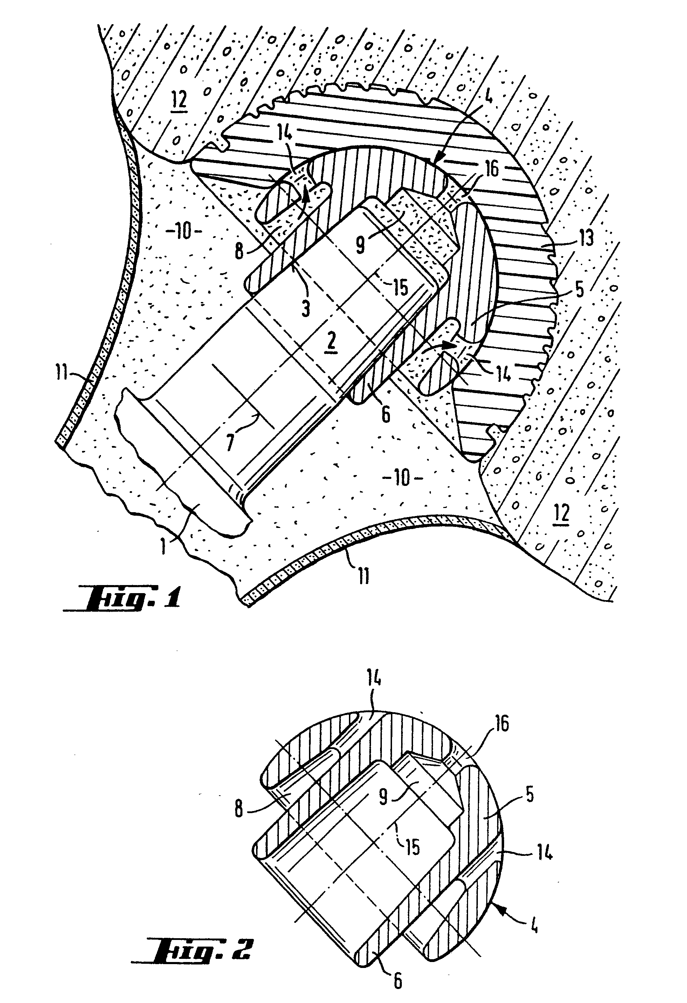

Um eine ausreichende Schmierung der aufeinander gleitenden Flächen von Gelenkkopf 4 und Gelenkpfanne 13 besonders in den »mittleren Breiten« der Kugelschale 5 sicherzustellen, sind erfindungsgemäß über den Umfang der Kugelschale 5 verteilte Durchtrittsöffnungen 14 vorgesehen, die den Ringraum 8 mit der »Lagerfläche« zwischen den Prothesenteilen verbinden. Diese Öffnungen können beispielsweise radial zur Symmetrieachse 15 der Konusschale 5 (Fig. 1) oder aber im wesentlichen parallel zu dieser Achse (Fig. 2) verlaufen. Selbstverständlich ist es auch möglich, die Öffnungen 14 mit einer Neigung zwischen diesen beiden Extremen anzuordnen.

Bei auf den Zapfen 2 aufgesteckten Gelenkkopf 4 verbleibt zwischen der Kugelschale 5 und dem Zapfen 2 im Innern der Konusbüchse 6 ein Hohlraum 9, der durch eine Bohrung 16 mit der Außenseite der Kugelschale 5 bzw. mit der Pfannenschale der Gelenkpfanne 13 in Verbindung steht; dieser Hohlraum 9, der ebenfalls Synovialflüssigkeit aufnimmt, kann als zusätzlicher Schmiermittelspeicher und/oder als Ablagerungsplatz für sich im Laufe der Zeit ansammelnden Abrieb dienen.

Der Gelenkkopf 4 besteht vorzugsweise aus einem der in der Implantat-Technik üblichen Metalle, einer Metall-Legierung oder aus Biokeramik, während der Zapfen im allgemeinen aus Metall, vorzugsweise aus einer für Implantate viel benützten CrNiMoCo-Legierung oder aber beispielsweise aus Titan gefertigt ist. Als Materialien für die Gelenkpfanne 13 dient in erster Linie Polyäthylen der Klassifikationen HDPE und UHMW, ohne daß andere gebräuchliche Pfannenwerkstoffe deswegen ausgeschlossen wären.

## Patentansprüche

1. Gelenkkopf (4) für eine Kugelgelenkprothese, der als Kugelschale (5) ausgebildet ist und schirmartig auf einem Stamm sitzt, wobei zwischen der Innenwand der Kugelschale und dem Stamm ein freier Ringraum (8) vorhanden ist, dadurch gekennzeichnet, daß im den Ringraum (8) begrenzenden Bereich der Kugelschale (5) über den Umfang verteilt Durchtrittsöffnungen (14) vorgesehen sind, die an die Außenseite der Kugelschale (5) führen.

2. Gelenkkopf nach Anspruch 1, wobei der Stamm (3) als Büchse (6) ausgebildet ist, die über einen Konus den Zapfen (2) eines Verankerungsschaftes (1) aufnimmt, dadurch gekennzeichnet, daß die Kugelschale (5) zusätzlich eine an sich bekannte zentrale Bohrung (16) aufweist, die eine offene Verbindung zu einem Hohlraum (9) im Innern der Büchse (6) herstellt.

3. Gelenkkopf nach Anspruch 1 oder 2, dadurch gekennzeichnet, daß die Durchtrittsöffnungen (14) parallel zu der Symmetrieachse (15) der Kugelschale (5) verlaufen.

## Claims

1. A ball (4) for a ball-and-socket joint prosthesis, the ball being in the form of a part-spherical member (5) disposed after the style of a mushroom on a stem (3), a free annular space (8) being left between the inner wall of the part-spherical member and the stem, characterised in that that zone of the part-spherical member (5) which defines the annular space (8) is formed with passage apertures (14) which are distributed over the periphery and which lead to the outside of the part-spherical member (5).

2. A ball for a ball-and-socket joint prosthesis according to claim 1, in which the stem (3) is in the form of a bush (6) which receives the peg (2) of an anchoring shank (1) by way of a cone, characterised in that the part-spherical member (5) additionally has a known central bore (16) which establishes an open connection to a cavity (9) inside the bush (6).

3. A ball for a ball-and-socket joint prosthesis according to claim 1 or 2, characterised in that the passage apertures (14) extend parallel to the axis of symmetry (15) of the part-spherical member (5).

## Revendications

1. Tête d'articulation (4) pour une prothèse à rotule, qui est réalisée sous la forme d'une calotte sphérique (5) et repose sur un fût à la manière d'un parapluie, un espace annulaire libre (8) se trouvant entre la paroi interne de la calotte sphérique et le fût, caractérisée par le fait que des orifices (14) de passage traversant, répartis sur le pourtour et prévus dans la zone de la calotte sphérique (5) délimitant l'espace annulaire (8), débouchent à la face externe de la calotte sphérique (5).

2. Tête d'articulation selon la revendication 1, dans laquelle le fût (3) est réalisé sous la forme d'une douille (6) recevant, par l'intermédiaire d'un cône, la cheville (2) d'une tige d'ancrage (1), caractérisée par le fait que la calotte sphérique (5) présente en plus un orifice central (16) connu en soi, qui établit une communication ouverte avec une cavité (9) à l'intérieur de la douille (6).

3. Tête d'articulation selon la revendication 1 ou 2, caractérisée par le fait que les orifices (14) de passage traversant s'étendent parallèlement à l'axe de symétrie (15) de la calotte sphérique (5).

**Fig. 1**

**Fig. 2**